# EUROPÄISCHE PATENTSCHRIFT

(11) **EP 1 363 496 B1**
(45) Veröffentlichungstag und Bekanntmachung des Hinweises auf die Patenterteilung: **22.09.2004**
(21) Anmeldenummer: 02748322.1
(22) Anmeldetag: 27.02.2002
(51) Int. Cl.: A01N 43/40

(54) **WUND- UND SCHLEIMHAUTANTISEPTIKUM**
ANTISEPTIC FOR WOUNDS AND MUCOUS MEMBRANES
ANTISEPTIQUE POUR BLESSURES ET MUQUEUSES

(30) Priorität: 01.03.2001 DE 10109925
(43) Veröffentlichungstag der Anmeldung: 26.11.2003
(73) Patentinhaber: Antiseptica Chemisch-Pharmazeutische Produkte GmbH, 50259 Pulheim (DE)
(72) Erfinder: KRAMER, Axel, 17489 Greifswald (DE); PITTEN, Frank-Albert, 17509 Wusterhusen (DE)
(74) Vertreter: Schaeffer, Michael
(86) Internationale Anmeldenummer: PCT/EP2002/002108
(87) Internationale Veröffentlichungsnummer: WO 2002/069874

(56) Entgegenhaltungen:
- EP-A- 0 411 315
- DE-A- 19 647 692
- US-A- 4 294 852
- US-A- 4 598 082

## Beschreibung

Die vorliegende Erfindung betrifft eine synergistische Wirkstoffkombination auf Basis von Octenidin zur antiseptischen Behandlung von Schleimhaut und/oder Wunden.

Octenidin mit der nachstehenden Formel 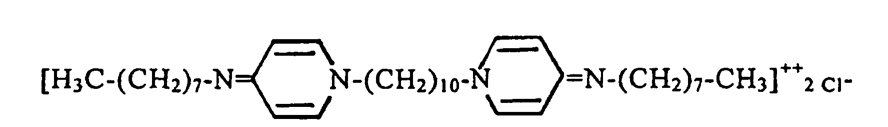 ist als Schleimhautantiseptikum bekannt und wird auch zur Wundantiseptik eingesetzt, allerdings wird diese Verbindung zur Erhöhung der Wirksamkeit regelmäßig mit Phenoxyethanol kombiniert. Ein derartiges Präparat ist beispielsweise unter der Marke "Octenisept" im Handel und wird vielfach in der Gynäkologie und Andrologie benutzt. Allerdings haben neuere Untersuchungen ergeben, daß die Kombination aus Octenidin und Phenoxyethanol eine hohe Zytotoxizität aufweist, so daß bei der Anwendung auf offenen Wunden beträchtliche Bedenken angebracht sind.

Weiterhin ist aus der DE 196 47 692 A1 bekannt, Octenidin als einen Wirkstoff bei der Händedesinfektion einzusetzen, wobei das Händedesinfektionsmittel Octenidin-Dihydrochlorid als Wirkstoff, einen C₁- bis C₈- Alkylalkohol, ein nichtionisches und/oder kationisches Tensid sowie gegebenenfalls eine α-Hydroxysäure enthält. Die speziellen Tenside werden eingesetzt, um das Desinfektionsmittel hautfreundlich zu gestalten.

Der Erfindung liegt die Aufgabe zugrunde, ein Wund- und Schleimhautantiseptikum auf der Basis von Octenidin zu entwickeln, das stabil ist und im Vergleich zu bekannten Mischungen über eine deutlich geringere Toxizität verfügt.

Zur Lösung der Aufgabe wird ein Wund- und Schleimhautantiseptikum mit einem Gehalt an Octenidin vorgeschlagen, das einen Gehalt an Ethanol und an einer physiologisch verträglichen organischen Säure aufweist, das dadurch gekennzeichnet ist, daß es Dicarbonsäuren oder Hydroxydi- oder -tricarbonsäuren enthält.

Völlig überraschend hat sich herausgestellt, daß durch die Kombination des Octenidin mit Ethanol in relativ geringen Mengen und Zugabe einer geringen Menge einer organischen Säure ein stabiles Wirkstoffgemisch mit synergistischer Wirksamkeit erzielt werden kann. Die Mischung enthält vorzugsweise 0,05 bis 0,1 Gew.-% Octenidin, 2 bis 10 Gew.-% Ethanol (94%) und die organische Säure in Mengen von etwa 0,5 bis 2 Gew.-%.

Als organische Säuren können Hydroxymonocarbonsäuren wie Milchsäure und Glycolsäure, Dicarbonsäuren wie Malonsäure oder Bernsteinsäure und gesättigte Hydroxydi- oder -tricarbonsäuren wie Äpfelsäure, Weinsäure oder Zitronensäure benutzt werden. Der pH der erfindungsgemäßen Antiseptika sollte im Bereich von etwa 2,5 bis 3,0, vorzugsweise bei 2,6 liegen, da dieser Bereich bei Anwendung auf Schleimhaut oder Wunden sich als günstig erwiesen hat.

Überraschenderweise sind die erfindungsgemäßen Mischungen klare farblose stabile Lösungen, die alle hervorragende Lagerfähigkeit aufweisen, was im Hinblick auf die Schwerlöslichkeit des Octenidin nicht zu erwarten war. Die Verträglichkeit auf Schleimhaut und offenen Wunden ist ebenfalls hervorragend, wobei noch zu bemerken ist, daß die Wirksamkeit im Vergleich zu einer Kombination von Octenidin und Phenoxyethanol oder im Verhältnis zu Octenidin als solchem in wäßriger Lösung deutlich gesteigert ist und einen echten Synergismus ergibt.

Die Erfindung wird anhand der Beispiele näher erläutert:

### Vergleichsbeispiel 1

Eine wäßrige Lösung mit einem Gehalt an *0,1* % Octenidin, 5 % Ethanol 94%-ig und 1,5 % Milchsäure 80%-ig wurde im quantitativen Suspensionstest untersucht. Eine Verdünnung mit 25 % dieser Mischung verursachte ohne Belastung bereits innerhalb von 30 Sekunden eine Verminderung von S. aureus und P. aeruginosa von über 5 log/Einheiten. Dasselbe Ergebnis wurde bei C. albicans nach 10 minütiger Einwirkung erhalten. Die 90%-ige Anwendungskonzentration der Mischung reduzierte C. albicans schon binnen 2,5 Minuten um mehr als 5 log/Einheiten. Unter massiver Albumin- sowie Blutbelastung von 10 % lagen die Reduktionen der Testbakterien durch die 50%-ige Anwendungskonzentration schon binnen 30 Sekunden Einwirkungszeit mit über 4,6 log/Einheiten über den von der DKH geforderten Werten von 3,0 log/Einheiten. Eine ausreichende Reduktion von c. albicans wurde unter diesen Belastungen mit derselben Konzentration während 4 Minuten verursacht. Selbst unter einer hohen Muzinbelastung von 10 % war die 90%-ige Anwendungskonzentration binnen 30 Minuten gegenüber S. aureus und P. aeruginosa effektiv.

Die Wirkstoffkombination ist gegenüber Bakterien und Hefen ein rasch wirkendes Produkt, das auch unter Belastung mit Albumin, Blut oder Muzin schnell und sehr effektiv wirkt.

### Beispiel 2

Im Explantattest (A. Kramer et al., Chirurg (1998) 60 : 840 - 845) wird das synergistische Wirkstoffgemisch gut toleriert. In 10%-iger Verdünnung wird eine Explantationsrate von 80 % mit einer Wachstumsrate von 60 % erreicht; im Vergleich dazu wird durch 10%-iges Octenisept bei der gleichen Einwirkungszeit von 30 Sekunden sowohl die Explantation als auch das Wachstum komplett unterdrückt.

Dieses Ergebnis war besonders überraschend, weil es keinerlei Hinweise auf die Aufhebung der Zytotoxizität durch die Kombination mit Ethanol und einer physiologisch annehmbaren organischen Säure gab.

### Beispiel 3

Bei diesem Test werden adhärente Kulturen von humanen Amnionzellen (FL-Zellen) über 24h bei 37°C mit der gewünschten Konzentration der Prüfsubstanz im kompletten MEM-Nährmedium mit 5% FBS in einer 5% CO₂/Luftatmosphäre inkubiert. Anschließend werden Nährmedium und Prüfsubstanz entfernt und neues Nährmedium mit Neutralrot zugegeben. Da vitale FL-Zellen den Farbstoff Neutralrot in ihre Lysosomen aufnehmen, korreliert die Intensität der eluierten Rotfärbung mit dem Anteil der lebenden Zellen. Die Intensität wird über die Extinktion bei 540 nm Messwellenlänge/655 nm Referenzwellenlänge automatisch bestimmt. Durch den Vergleich mit einer Kontrollgruppe, die anstelle der Prüfsubstanz mit phosphatgepufferter Kochsalzlösung (PBS) inkubiert wurde ist eine Aussage über die zytotoxische Wirkung der Prüfsubstanz möglich. Des weiteren können unterschiedliche Formulierungen an diesem Modell hinsichtlich ihrer Zytotoxizität untersucht und verglichen werden.

In der vorliegenden Untersuchung wurden unterschiedliche Konzentrationen von Octenidindihydrochlorid in PBS und Octenisept® (enth. Octenidindihydrochlorid und Phenoxyethanol) miteinander und gegenüber PBS verglichen. Die Konzentrationsangaben beziehen sich jeweils auf die Konzentration von Octenidindihydrochlorid.

### Ergebnis:

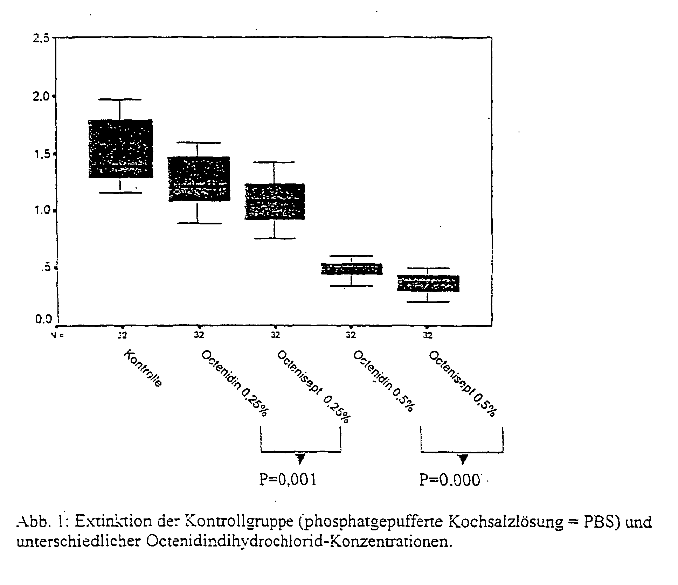

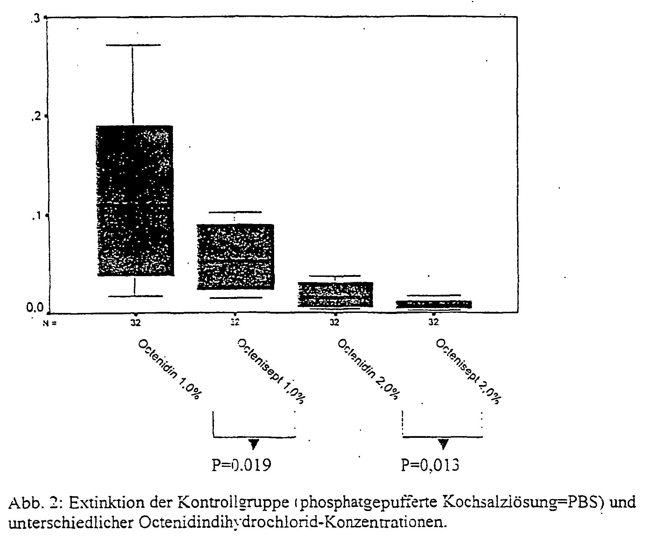

Die ermittelten Extinktionen sind in Abb. 1-2 widergegeben. Jede Messung beruht auf 32 Einzelbestimmungen; zur Inferenzstatistik wurde der U-Test verwendet. Es zeigt sich, daß bei allen geprüften Konzentrationen die Extinktion der Reinsubstanz Octenidindihydrochlorid gegenüber der des Fertigpräparates Octenisept® signifikant erhöht ist. Dies bedeutet, daß die zytotoxische Wirkung von Octenidindihydrochlorid bei vergleichbarer Konzentration dieses Wirkstoffes im Fertigarzneimittel Octenisept® signifikant geringer ist. Ursache für diesen additiven zytotoxischen Effekt kann nur die Kombination von Octenidindihydrochlorid im Octenisept® mit anderen Substanzen, am ehesten mit Phenoxyethanol, sein.

## Patentansprüche

1. Wund- und Schleimhautantiseptikum auf Basis von Octenidin mit einem Gehalt an Ethanol und einer physiologisch verträglichen organischen Säure, **dadurch gekennzeichnet, daß** es Dicarbonsäuren oder Hydroxydi- oder -tricarbonsäuren enthält.

2. Wund- und Schleimhautantiseptikum nach Anspruch 1, **gekennzeichnet durch** einen Gehalt an einer Säure aus der Gruppe Glycolsäure, Malonsäure, Bersteinsäure, Apfelsäure, Weinsäure oder Zitronensäure.

3. Wund- und Schleimhautantiseptikum nach Anspruch 1 oder 2, **gekennzeichnet durch** einen Gehalt an 0,05 bis 0,1 Gew.-% Octenidin, 2 bis 20 Gew.-% Ethanol und 0,5 bis 2,5 Gew.-% einer Dicarbonsäure oder Hydroxydi- oder -tricarbonsäure.

## Claims

1. An antiseptic for wounds and mucuous membranes, based on octenidine with a content of ethanol and a physiologically compatible organic acid, **characterised in that** it contains dicarboxylic acids or hydroxydi- or tricarboxylic acids.

2. An antiseptic for wounds and mucuous membranes according to claim 1, **characterised by** a content of an acid from the group comprising glycolic acid, malonic acid, succinic acid, malic acid, tartaric acid or citric acid.

3. An antiseptic for wounds and mucuous membranes according to claim 1 or 2, **characterised by** a content of 0.05 to 0.1 wt.% of octenidine, 2 to 20 wt.% of ethanol and 0.5 to 2.5 wt.% of a dicarboxylic acid or hydroxydi- or tricarboxylic acid.

## Revendications

1. Antiseptique des plaies et des muqueuses, à base d'octénidine et contenant de l'éthanol et un acide organique physiologiquement compatible, **caractérisé en ce qu'**il contient un acide dicarboxylique, hydroxy-dicarboxylique ou hydroxy-tricarboxylique.

2. Antiseptique des plaies et des muqueuses, conforme à la revendication 1, **caractérisé en ce qu'**il contient un acide choisi dans l'ensemble que constituent les acides glycolique, malonique, succinique, malique, tartrique et citrique.

3. Antiseptique des plaies et des muqueuses, conforme à la revendication 1 ou 2, **caractérisé en ce qu'**il contient de 0,05 à 0,1 % en poids d'octénidine, de 2 à 20 % en poids d'éthanol et de 0,5 à 2,5 % en poids d'un acide dicarboxylique, hydroxy-dicarboxylique ou hydroxy-tricarboxylique.
